(19) Europäisches Patentamt
European Patent Office
Office européen des brevets

(11) Veröffentlichungsnummer: **0 175 263 B1**

# (12) EUROPÄISCHE PATENTSCHRIFT

(45) Veröffentlichungstag der Patentschrift: **05.06.91**

(51) Int. Cl.5: **C07D 241/20**, C07D 403/04

(21) Anmeldenummer: **85111442.1**

(22) Anmeldetag: **10.09.85**

Die Akte enthält technische Angaben, die nach dem Eingang der Anmeldung eingereicht wurden und die nicht in dieser Patentschrift enthalten sind.

## (54) 2-Amino-3-chlormethyl-5-(dichlormethyl)-pyrazine und Verfahren zu deren Herstellung.

(30) Priorität: **15.09.84 DE 3433959**

(43) Veröffentlichungstag der Anmeldung:
**26.03.86 Patentblatt 86/13**

(45) Bekanntmachung des Hinweises auf die Patenterteilung:
**05.06.91 Patentblatt 91/23**

(84) Benannte Vertragsstaaten:
**CH DE FR GB LI NL**

(56) Entgegenhaltungen:
**US-A- 4 160 834**

**BARLIN, THE PYRAZINES (John Wiley 1982) S. 114**

**JOURNAL OF ORGANIC CHEMISTRY, Vol. 43, No. 4 (1978), S. 736**

(73) Patentinhaber: **BASF Aktiengesellschaft**
**Carl-Bosch-Strasse 38**
**W-6700 Ludwigshafen(DE)**

(72) Erfinder: **Leininger, Hartmut, Dr.**
**Dr.-Siebenpfeiffer-Strasse 22**
**W-6730 Neustadt(DE)**
Erfinder: **Littmann, Wolfgang, Dr.**
**Neckarpromenade 36**
**W-6800 Mannheim 1(DE)**
Erfinder: **Paust, Joachim, Dr.**
**Ringstrasse 3**
**W-6708 Neuhofen(DE)**
Erfinder: **Trautmann, Walter, Dr.**
**Ritterbueschel 87**
**W-6730 Neustadt(DE)**

**EP 0 175 263 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft 2-Amino-3-chlormethyl-5-dichlormethyl-pyrazine der allgemeinen Formel I

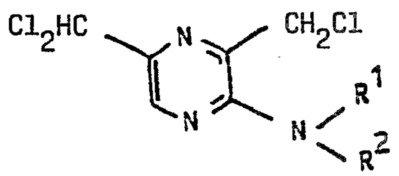

(I)

in welcher $R^1$ und $R^2$ für Wasserstoff oder eine Schutzgruppe stehen, wobei $R^1$ und $R^2$ auch miteinander verbunden sein können, vorzugsweise solche Pyrazine der Formel I, in der $R^1$ und $R^2$ zusammen einen Phthaloylrest bedeuten.

Außerdem betrifft die Erfinduug ein Verfahren zur Herstellung der Verbindungen I.

Der Erfinduug lag die allgemeine Aufgabe zugrunde, die physiologisch wichtige Folsäure

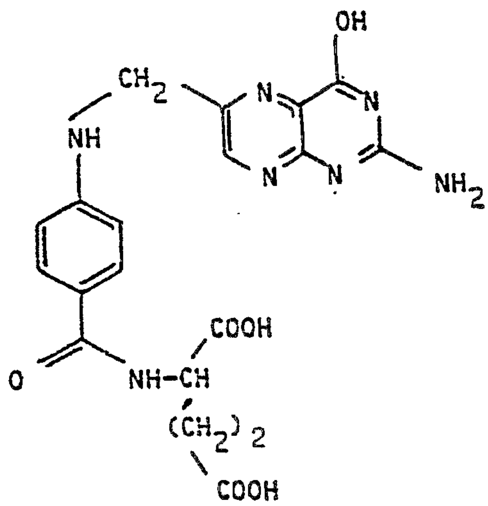

sowie deren Derivate besser zugänglich zu machen. Speziell betraf die Aufgabe neue Zwischenprodukte mit der 2-Amino-pyrazin-Struktur, die einerseits gut zugänglich sind und sich andererseits auf relativ einfache Weise in reaktionsfähige Pteridine und weiter in die Folsäure bzw. deren Derivate überführen lassen.

Aus einer Arbeit von E.C. Taylor et al (J. Org. Chem., Vol. 43 (1978) No. 4, Seite 736) ist es bekannt, daß man 6-Folmyl-pterin der Formel

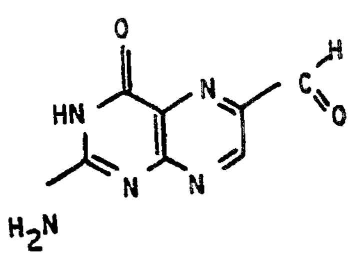

welches eine Schlüsselverbindung für Folsäure und die verschiedensten Derivate davon darstellt, auf relativ einfache Weise und mit guten Ausbeuten aus 2-Amino-3-cyano-5-dialkoxymethyl-pyrazin herstellen kann. Die gemäß dieser Arbeit für die Herstellung des 2-Amino-3-cyano-5-dialkyoxymethyl-prazins vorgesehene

Ausgangsverbindung, das 2-Amino-3-cyano-5-chlormethyl-pyrazin ist jedoch leider selbst nicht gut zugänglich. Man erhält es beispielsweise in nur mäßigen Ausbeuten aus Aminomalonitril und ß-Chloro-pyruvaldoxim, welches seinerseits aus Keten hergestellt werden kann und eine physiologisch äußerst unangenehme Substanz ist, die bei Raumtemperatur unter Bildung von Blausäure, zerfällt und daher für eine technische Umsetzung ungeeignet ist (vgl. E.C. Taylor et al, J. Org. Chem. 38 (1973), 806). Trotz großer Anstrengungen des Arbeitskreises um E.C. Taylor (vgl. J. Org. Chem. 45, (1980) Seiten 2485 bis 2489 und J. Org. Chem. 46 (1981), Seiten 1394 bis 1402) ist es nicht gelungen, ein technisch brauchbares Verfahren zur Herstellung von reinen isomerenfreien 2-Amino-3-cyano-5-dialkoxymethyl-pyrazinen zu entwickeln, so daß die 2-Amino-3-cyano-5-dialkoxymethyl-pyrazine bislang nur als schwer zugängliche, in schlechten Ausbeuten, in zahlreichen Stufen und/oder aus teuren und nicht gut synthetisierbaren Ausgangsmaterialien herstellbare Substanzen bekannt waren.

Es war daher die Aufgabe der Erfindung, neue Zwischenprodukte mit der 2-Amino-pyrazin-Struktur zu finden, die einerseits gut zugänglich sind und andererseits auf relativ einfache Weise in 2-Amino-3-cyano-5-dialkoxymethyl-pyrazin überführt werden können, wodurch ein vorteilhaftes Gesamtverfahren zur Herstellung von Folsäure und davon abgeleiteten Verbindungen über 6-Formyl-pterin ermöglicht würde.

Es wurde nun überraschender Weise gefunden, daß man in guten Ausbeuten 2-Amino-3-chlormethyl-5-dichlormethyl-pyrazine der allgemeinen Formel I erhält, wenn man die entsprechenden 2-Amino-3,5-dimethyl-pyrazine der allgemeinen Formel II

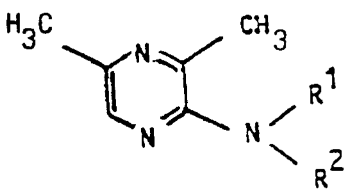

(II),

in der $R^1$ und $R^2$ Schutzgruppen bedeuten,
mit Chlor behandelt und die Schutzgruppen nach beendeter Chlorierung gewünschtenfalls wieder abspaltet.

Der Stammkörper der Ausgangsverbindungen II ($R^1$ = $R^2$ = H) ist bekannt und nach dem Verfahren der DE-A-32 42 195 auf relativ einfache Weise durch Cyclisierung von α-Imino-dipropionsäurenitril der Formel III

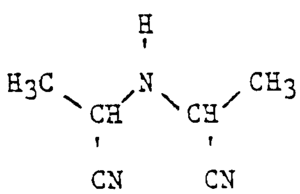

(III)

mit Halogenwasserstoffen erhältlich. III seinerseits kann durch die in DE-A-14 93 752 beschriebene Umsetzung von Ammoniak mit Acetaldehyd und Blausäure hergestellt werden.

Andererseits können die erfindungsgemäßen 2-Amino-3-chlormethyl-5-dichlormethyl-pyrazine der Formel I, beispielsweise gemäß dem Verfahren einer gleichzeitig eingereichten Patentanmeldung (EP-A-175 264) auf relativ einfache und vorteilhafte Weise in wertvolle Vorprodukte der Folsäure bzw. von Folsäurederivaten überführt werden.

Daß die erfindungsgemäße Chlorierung der 2-Amino-3,5-dimethyl-pyrazine II zu den Verbindungen der Formel I mit so guten Ausbeuten gelingt,war nicht zu erwarten, sondern war überraschend. Das ergibt sich überzeugend, wenn man sich die Ausführungen von Standardwerken der Chemie über das Problem der Halogenierung von Stickstoff enthaltenden Heterocyclen ansieht. So heißt es beispielsweise in Houben-Weyl, "Methoden der organischen Chemie, Band 5/3, Georg-Thieme Verlag Stuttgart, 1962, auf Seite 728 zur Kernchlorierung: "Die Chlorierung von Pyridinen und Pyridin-Derivaten führt meist mit geringen Ausbeuten zu höher chlorierten Produkten die mit steigender Temperatur einen wachsenden Anteil an teerartigen Bestandteilen enthalten". Dies würde bedeuten, daß man a priori mit Nebenreaktionen wie Kernchlorierung, aber auch mit unkontrollierten Weiterreaktionen zu rechnen hätte. Weiter heißt es auf Seite

731 "Pyrazin kann bei Raumtemperatur nicht chloriert werden, dagegen aber bei höheren Temperaturen", wobei sich für das angemeldete Verfahren die Frage erhob, welchen Einfluß die verschiedenen Substituenten auf die Halogenierung nehmen könnten.

Weiter kann bezüglich der Ausführungen über die Seitenkettenhalogenierung von Aromaten die Seite 748 genannt werden, in der es heißt: "Die direkte Chlorierung von ß-Picolin, 3-Methyl-chinolin, Lepidin, 1-, 3- und 4-Methyl-isochinolin in der Seitenkette ist nicht möglich", und die Chlorierung von Picolin betreffend: "Da die Chlorierung sofort bis zur Trichlorstufe verläuft ..." und "α-Picolin und α-Picolin sind in der Seitenkette chlorierbar, aber nicht bromierbar". Aus allem ergibt sich, daß der Durchschnittsfachmann annehmen mußte, daß sowohl die Halogenierung an sich, die Chlorierung wie auch die Bromierung im besonderen, überhaupt nicht gelingen würde oder aber lediglich zu perchlorierten Produkten führt. Zusätzlich müßten bei Verwendung der Verbindungen der Formel II als Ausgangsverbindungen Komplikationen in Bezug auf die Nichtäquivalenz der beiden Methylgruppen befürchtet werden, denn aus ibid. Seite 748) war folgendes bekannt: "Aus 3,4,5-Trimethyl-pyrazol entsteht bei der Chlorierung in Eisessig 4-Methyl-3,5-bis-trichlormethyl-pyrazol in 74 %iger Ausbeute. Im 1,3,5-Trimethyl-pyrazol dagegen wird ... nur eine Methylgruppe substituiert."

Es war zwar aus "The Pyrazines" von G.B. Barlin, John Wiley & Sons, New York, Chichester, Brisbane, Toronto, Singapore, 1982, schon bekannt, einige Methylpyrazine und Dimethylpyrazine zu chlorieren, jedoch wurden bei der Chlorierung der ähnlichsten Verbindung, dem 2,5-Dimethylpyrazin, unter ähnlichen Bedingungen im wesentlichen das Kernchlorierungsprodukt 3-Chloro-2,5-dimethyl-pyrazin erhalten.

Zusammenfassend kann man sagen, daß auf Grund des Standes der Technik nicht erwartet werden konnte, daß die 2-Amino-3,5-dimethyl-pyrazine II durch Chlorierung in so guten Ausbeuten in die 2-Amino-3-chlormethyl-5-dichlormethyl-pyrazine der Formel I überführt werden können, so daß damit reine und isomerenfreie Vorprodukte für Pteridinsynthesen, wie sie sonst nur äußerst schwierig und bisher nicht im technischen Maßstab synthetisiert werden könnten, technisch zugänglich werden.

Für die Chlorierung empfiehlt es sich zumeist, die Aminogruppe des 2-Amino-3,5-dimethyl-pyrazins in an sich bekannter Weise zu schützen. Als Schutzgruppen können die allgemein üblichen, auch aus der Peptidchemie bekannten Gruppen, wie die Carbobenzyloxy-, Carbo-tert.-butyloxy-, Triphenylmethyl-, p-Toluolsulfonyl-, Formyl-, Trifluoracetyl-, die Carbo-p-nitrobenzyloxy-und die Phthaloylgruppe dienen.

Besonders gut eignet sich die o-Phthalsäure zum Schutze der 2-Aminogruppe, wobei sich die gegen Oxidationen chemisch sehr stabile Phthalimid-Gruppierung ausbildet.

Zur Einführung dieser Schutzgruppe setzt man die freie 2-Amino-Verbindung II zweckmäßigerweise bei 80 bis 120° C in Gegenwart eines inerten Lösungsmittels mit Phthalsäureanhydrid um, wobei man das hierbei abgespaltene Wasser durch Azeotropdestillation mit dem Lösungsmittel laufend aus dem Reaktionsgemisch entfernt. Als LÖsungsmittel kommen hierfür z.B. Benzol, Toluol, die Xylole und Chlorbenzol in Betracht.

Auf analoge Weise kann man die Aminogruppe auch mit anderen, vorzugsweise mit starken Carbon- oder Sulfonsäuren einfach oder zweifach acylieren, z.B. mit dem tert.-Butylester, dem Benzylester oder dem p-Nitro-benzylester der Chlorkohlensäure, der Ameisensäure, der Trifluoressigsäure oder der p-Toluolsulfonsäure.

Auch andere Schutzgruppen, wie die Triphenylmethylgruppe, die man durch Umsetzung von Triphenylmethylchlorid mit der freien Aminoverbindung II erhält, kommen in Betracht. Auch die freie Aminoverbindung II kann man dem Verfahren unterwerfen, jedoch ist hierbei mit Ausbeuteverlusten von I infolge von Nebenreaktionen zu rechnen.

Die Chlorierung erfolgt nach den bekannten Gesetzen der Seitenketten-Halogenierung aromatischer Verbindungen, vorzugsweise in Gegenwart eines inerten Lösungsmittels wie Tetrachlorkohlenstoff oder Chlorbenzol bei 20 bis 132° C; - vorzugsweise unter Erhitzen auf die Siedetemperatur des Gemisches unter Rückfluß des Lösungsmittels.

Da die Seitenkettenhalogenierung bekanntlich vornehmlich über einen radikalischen Mechanismus verläuft, empfiehlt sich zur Beschleunigung und Vervollständigung der Reaktion die Mitverwendung katalytischer Mengen von Radikalbildnern wie Benzoylperoxid oder Azodiisobutyronitril. Anstelle der Radikalbildner oder zusätzlich zu diesen kann man auch UV-Licht auf das Reaktionsgemisch einwirken lassen.

Als Chlorierungsprodukte erhält man im Grenzfall ein Gemisch aus u.U. 16 verschiedenen Chlorverbindungen. Für Verbindungen der Formel I, in der $R^1$ und $R^2$ für H oder $R^1$ und $R^2$ zusammen für die Phthaloylgruppe stehen, seien genannt: 5-Chlormethyl-3-methyl-2-phthalimidopyrazin, 3-Chlormethyl-5-methyl-2-phthalimido-pyrazin, 3,5-Bis-(chlormethyl)-2-phthalimido-pyrazin, 5-Dichlormethyl-3-methyl-2-phthalimido-pyrazin, 3-Dichlormethyl-5-methyl-2-phthalimido-pyrazin, 5-Chlormethyl-3-dichlormethyl-2-phthalimido-pyrazin, 3-Chlormethyl-5-dichlormethyl-2-phthalimido-pyrazin, 3,5-Bis(dichlormethyl)-2-phthalimido-pyrazin, 3-Methyl-2-phthalimido-5-trichlormethyl-pyrazin, 3-Chlormethyl-2-phthalimido-5-trichlormethyl-pyrazin, 3-Dichlormethyl-2-phthalimido-5-trichlormethyl-pyrazin bzw. die entsprechenden ungeschützten 2-Amino-Analogen.

Bei den Chlorierungen können aber auch einzelne Reaktionsprodukte bevorzugt synthetisiert werden. Dies geschieht beispielsweise dadurch, daß bei Durchführung des erfindungsgemäßen Verfahrens die für das gewünschte Produkt optimale Stöchiometrie verwendet wird, oder aber dadurch, daß die Reaktion unter Verwendung eines Überschusses an Halogenierungsmittel zum optimalen Zeitpunkt abgebrochen wird.

Bemerkenswerterweise fällt unter den Chlorierungsprodukten dasjenige besonders ins Gewicht, das der Formel I entspricht, wenn man die Reaktionsbedingungen, Menge des Halogenierungsmittels bzw. Reaktionsdauer optimal erstellt.

Im Hinblick auf die weitere Umsetzung hat die 3-Monochlormethyl-5-dichlormethyl-Verbindung der Formel I die größte Bedeutung, so daß man es aufgrund der oben genannten Gesetzmäßigkeit in der Hand hat, die Halogenierung gerade auf dieser Stufe - etwa durch Abkühlen - abzubrechen, bevor der durchschnittliche Halogenierungsgrad der beiden Methylgruppen noch weiter ansteigt.

Wesentlich ist lediglich, die Reaktion auf der Stufe des relativ größten Anteils von I abzubrechen. Dieser Anteil läßt sich in Vorversuchen z.B. durch Kernresonanz- oder HPLC-Analyse leicht bestimmen. Ist die optimale Reaktionszeit erst einmal auf diese Weise ermittelt worden, bedarf es für die Ausführung des Verfahrens in technischem Maßstab keiner derartigen analytischen Kontrolle mehr. Auf diese Weise lassen sich Ausbeuten von 69 %, bezogen auf II, an den besonders interessanten Verbindungen der Formel I erzielen.

Wegen der hohen Anteile an Verbindungen der Formel I bereitet die Aufarbeitung der Reaktionsgemische auf diese Produkte keine Schwierigkeiten, da sie aus praktisch allen Lösungsmitteln bevorzugt kristallisieren. Die übrigen Verbindungen können dann, falls gewünscht, in üblicher, wenn auch etwas weniger einfacher Weise, aus den Mutterlaugen durch Kristallisation gewonnen werden.

Als Lösungsmittel für die Kristallisation eignen sich u.a. $C_1$-$C_4$-Alkanole, z.B. Isopropanol, Ketone wie Aceton, halogenierte Kohlenwasserstoffe wie Methylenchlorid, aromatische Kohlenwasserstoffe wie Toluol, sowie Acetonitril. Bereits nach einmaliger Kristallisation erhält man das Hauptprodukt der Formel I in einer Reinheit von über 80%, wie es für die meisten weiteren Umsetzungen normalerweise ausreicht. Höhere Reinheiten können wie üblich durch weitere Umkristallisationsvorgänge erzielt werden.

Häufig reicht ein Reinheitsgrad von 60 bis 80 % jedoch aus, weil sich die Folgeprodukte von I in der Regel leichter von den übrigen Komponenten des Umsetzungsgemisches abtrennen lassen als I selber von den anderen Chlorierungsprodukten.

Für Spezialzwecke oder für analytische Zwecke lassen sich sämtliche Chlorierungsprodukte durch Säulenchromatographie in hochreiner Form gewinnen. Vorzugsweise verwendet man hierzu als feste Phase Kieselgel mit der Korngröße von 0,02 bis 0,2 mm Durchmesser und als flüssige Trägerphase ein Gemisch aus 5 Vol.-Teilen 1,2-Dichlorethan und 1 Vol.-Teil Ethylacetat.

Die Schutzgruppen von I können vor oder nach der Reindarstellung durch Kristallisation oder Chromatographie in an sich bekannter Weise, z.B. durch Umsetzung mit Hydrazin abgespalten werden.

Da die Verbindungen I normalerweise jedoch für weitere Synthesen verwendet werden, in denen sich der Schutz der 2-Aminogruppe ebenfalls empfiehlt, wird man die Schutzgruppen in der Regel nicht abspalten.

Über die Verbindungen I sind durch übliche Substitution des Halogenrestes die entsprechenden Verbindungen enthaltend eine ($-CH_2-OH$)-, ($-CHO$)-und ($-COOH$)-Gruppe sowie die davon abgeleiteten Ether, Acetale, Nitrile und Ester zugänglich. So gelangt man auf folgendem Wege von I zu dem Pteridin-Baustein der Folsäure:

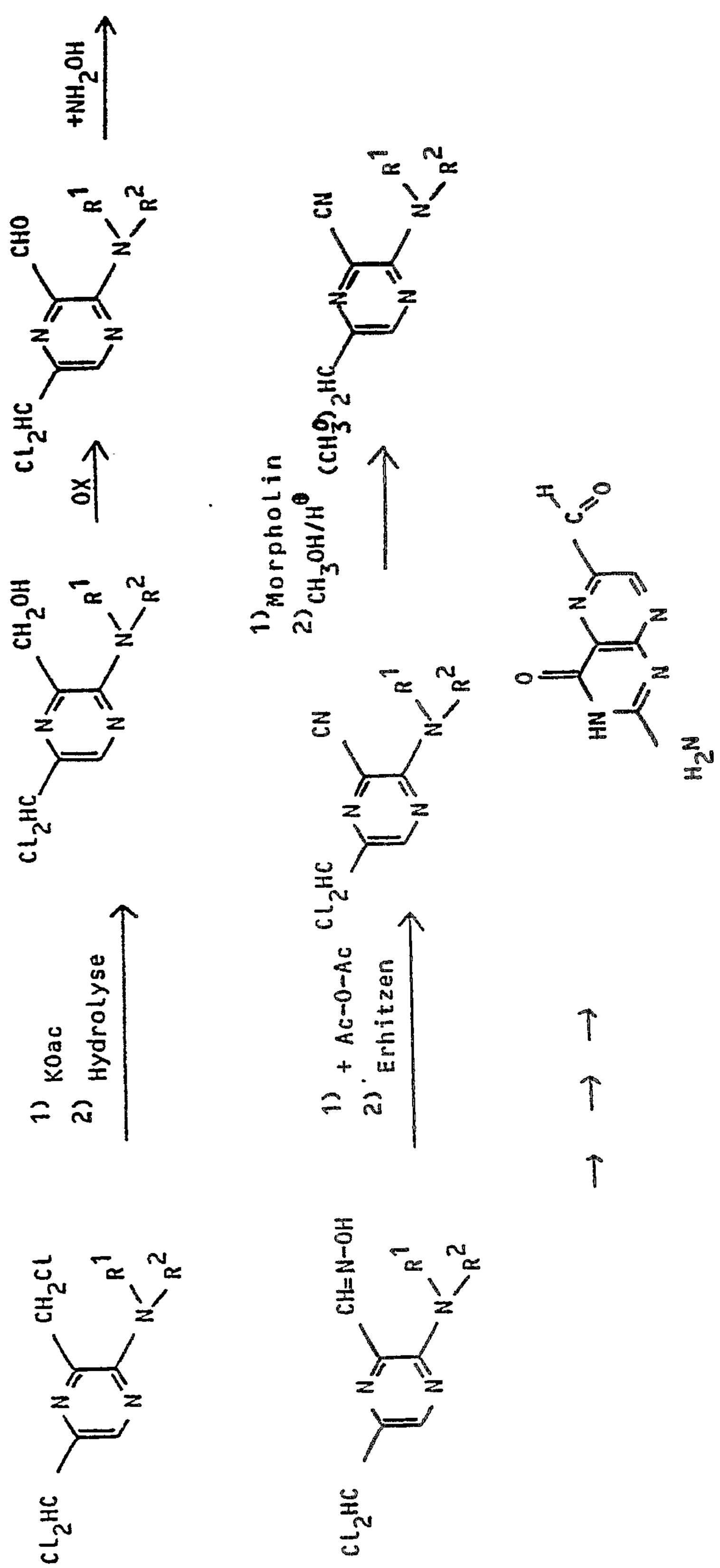


Beispiel

a) Herstellung von 3,5-Dimethyl-2-phthalimido-pyrazin

Eine Lösung aus 307 g 2-Amino-3,5-dimethyl-pyrazin und 1100 g Chlorbenzol wurde bei 25° C mit 481 g

Phthalsäureanhyrid versetzt und anschließend unter laufender Entfernung des Reaktionswassers 6 h zum Sieden erhitzt.

Nach beendeter Umsetzung wurde zu der erhaltenen braunen Lösung 1 1 Wasser addiert, wonach das Chlorbenzol unter Konstanthaltung des Wasservolumens zusammen mit Wasser abdestilliert wurde. Danach wurde die verbleibende wäßrige Lösung nochmals mit 0,5 1 Wasser versetzt, zum Sieden erhitzt und auf 25 bis 30 °C abgekühlt, wobei das Produkt ausfiel. Das erhaltene Gemisch wurde noch 2 h bei dieser Temperatur gerührt, wonach das Produkt abgetrennt, in 1,5 1 siedendem Methanol aufgenommen und nach Abkühlen auf 0 °C abfiltriert wurde. Die Ausbeute war praktisch quantitativ, der Schmelzpunkt F = 142 bis 143 °C.

b) Chlorierung von 3,5-Dimethyl-2-phthalimido-pyrazin

Eine Lösung von 63,2 g (0,25 mol) des gemäß a) erhaltenen Pyrazines, in 250 ml Chlorbenzol wurde bei 95 °C mit 7,5 ml einer Lösung von 0,42 g Azodiisobutyronitril in Chlorbenzol versetzt und in das Reaktionsgemisch im Laufe von 4 h bei 100 °C 107 g (3 val) Chlor eingeleitet, wobei nach 2,5 bis 3,5 h jeweils weitere 0,42 g Azodiisobutyronitril zugesetzt wurden.

Danach wurde das Chlorbenzol bei 80 °C abdestilliert und der Rückstand aus 260 ml Isopropanol umkristallisiert. Die Ausbeute an den Chlorierungsprodukten betrug 69 g.

Wie durch Kernresonanzspektroskopie und HPLC-Analyse ermittelt wurde, bestand das Gemisch zu

42 % aus 3-Chlormethyl-5-dichlormethyl-2-phthalimido-pyrazin

29 % aus 3,5-Bis-(dichlormethyl)-2-phthalimido-pyrazin

21 % aus 3-Dichlormethyl-5-chlormethyl-2-phthalimido-pyrazin sowie zu insgesamt

8 % aus den Verbindungen 3-Dichlormethyl-5-trichlormethyl-2-phthalimido-pyrazin, 3-Methyl-5-dichlormethyl-2-phthalimido-pyrazin, 3,5-Bis(chlormethyl)-2-phthalimido-pyrazin und 3-Methyl-5-chlormethyl-2-phthalimido-pyrazin.

Das kristalline Rohprodukt wurde in 130 ml Aceton heiß aufgenommen. Nach Abkühlen erhielt man 33,0 g eines Produktgemisches mit einem Ansteil von 62 % an 3-Chlormethyl-5-dichlormethyl-2-phthalimido-pyrazin. Umkristallisation aus Dichlormethan erhöhte bei einer Ausbeute von 14,3 g den Anteil an 3-Chlormethyl-5-dichlormethyl-2-phthalimido-pyrazin auf 80 %.

Die Mutterlauge der Acetonbehandlung wurde mit Toluol versetzt, von Aceton befreit und auf Raumtemperatur (RT) abgekühlt. Man erhielt 18,3 g eines Produktgemisches mit einem Anteil an 3,5-Bis-(dichlormethyl)-2-phthalimido-pyrazin von 60 %.

## Ansprüche

1. 2-Amino-3-chlormethyl-5-dichlormethyl-pyrazine der allgemeinen Formel I

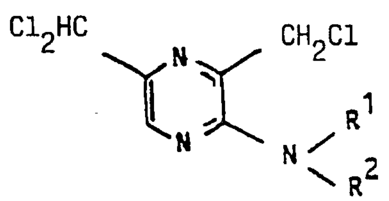

(I),

in welcher $R^1$ und $R^2$ für Wasserstoff oder eine Schutzgruppe stehen, wobei $R^1$ und $R^2$ auch miteinander verbunden sein können.

2. 2-Amino-3-chlormethyl-5-dichlormethyl-pyrazin der allgemeinen Formel I gemäß Anspruch 1, in der $R^1$ und $R^2$ zusammen einen Phthaloylrest bedeuten.

3. Verfahren zur Herstellung der 2-Amino-3-chlormethyl-5-dichlormethyl-pyrazine der allgemeinen Formel I gemäß Anspruch 1, dadurch gekennzeichnet, daß man 2-Amino-3,5-dimethyl-pyrazine der allgemeinen Formel II

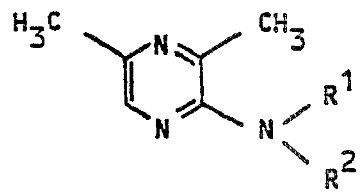

(II),

in der $R^1$ und $R^2$ Schutzgruppen bedeuten,
mit Chlor behandelt, dei Reaktion auf der Stufe des größten Anteils der Verbindung des Formel I abbricht und die Schutzgruppen nach beendeter Chlorierung gewünschtenfalls wieder abspaltet.

## Claims

1. A 2-amino-3-chloromethyl-5-dichloromethylpyrazine of the formula I

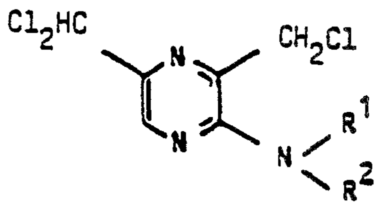

(I)

where $R^1$ and $R^2$ are each hydrogen or a protective group or may furthermore be bonded to one another.

2. A 2-amino-3-chloromethyl-5-dichloromethylpyrazine of the formula I as claimed in claim 1, in which $R^1$ and $R^2$ together are phthaloyl.

3. A process for the preparation of a 2-amino-3-chloromethyl-5-dichloromethylpyrazine of the formula I as claimed in claim 1, wherein a 2-amino-3,5-dimethylpyrazine of the formula II

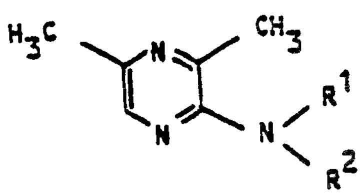

(II)

where $R^1$ and $R^2$ are protective groups, is treated with chlorine, the reaction is interrupted at the stage when the largest proportion of the compound of the formula I is present and the protective groups are, if desired, eliminated when the chlorination is complete.

## Revendications

1. 2-Amino-3-chloromethyl-5-dichloromethylpyrazine de formule générale I

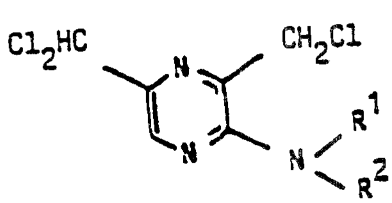

(I),

dans laquelle $R^1$ et $R^2$ sont mis pour des atomes d'hydrogène ou des groupements protecteurs. $R^1$ et $R^2$ pouvant aussi être liés l'un à l'autre.

**2.** 2-Amino-3-chlorométhyl-5-dichlorométhylpyrazine de formule générale I selon la revendication 1, dans laquelle $R^1$ et $R^2$ représentent ensemble un reste phtaloyle.

**3.** Procédé de préparation de la 2-amino-3-chlorométhyl-5-dichlorométhylpyrazine de formule générale 1 selon la revendication 1, caractérisé en ce qu'on traite par le chlore une 2-amino-3,5-diméthylpyrazine de formule générale II

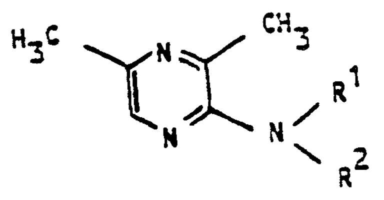

(II),

dans laquelle $R^1$ et $R^2$ représentent des groupements protecteurs, on arrête la réaction à l'obtention de la plus grande proportion du composé de formule I et on coupe les groupements protecteurs, si on le désire, une fois la chloration terminée.